# FASCICULE DE BREVET EUROPEEN

(11) **EP 0 576 330 B1**
(45) Date de publication et mention de la délivrance du brevet: **27.09.1995**
(21) Numéro de dépôt: 93401555.3
(22) Date de dépôt: 17.06.1993
(51) Int. Cl.: C07J 41/00

(54) **Nouveau procédé de préparation d'un composé stéroide delta 9(11)**
Neues Verfahren zur Herstellung Delta-9(11)-Steroide
A new process for producing 9(11)-unsaturated steroids

(30) Priorité: 19.06.1992 FR 9207460
(43) Date de publication de la demande: 29.12.1993
(73) Titulaire: ROUSSEL UCLAF, 93230 Romainville (FR)
(72) Inventeur: Buendia, Jean, F-94170 Le Perreux Sur Marne (FR); Roussel, Patrick, F-94320 Thiais (FR); Vivat, Michel, F-77400 Lagny Sur Marne (FR)
(74) Mandataire: Vieillefosse, Jean-Claude

(56) Documents cités:
- EP-A- 0 263 569
- EP-A- 0 294 911
- FR-A- 2 387 245
- STEROIDS: STRUCTURE, FUNCTION, AND REGULATION vol. 55, no. 3, Mars 1990, STONEHAM, MA US pages 109 - 113 J. N. M. BATIST ET AL 'The Chemistry of 9-alpha-Hydroxysteroids. 3. Methods for Selective Formation and Dehydrations of 17 -beta-cyano-9-alpha,17-alpha-dihydroxyandr osta-4-en-3-one'

## Description

La présente invention concerne un nouveau procédé de préparation d'un composé stéroïde Δ9(11).

L'invention a ainsi pour objet un procédé industriel de préparation du composé de formule (I) : 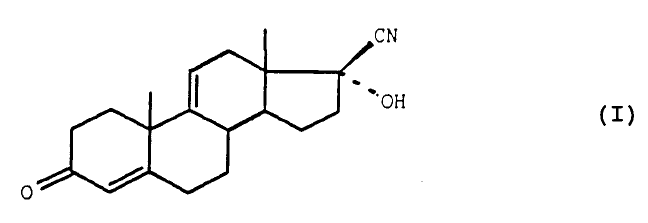
à partir d'un composé de formule (II): 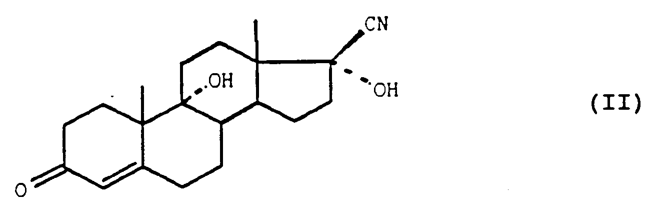
caractérisé par le fait que l'on traite le composé de formule par l'acide chlorosulfonique au sein d'un solvant organique.

Dans des conditions préférentielles de mise en oeuvre du procédé de l'invention, on opère au sein d'un solvant aprotique polaire choisi dans le groupe constitué par le tétrahydrofuranne, le dioxanne et l'acétone, ou de l'acétate d'éthyle ou du chlorure de méthylène, le tétrahydrofuranne et l'acétone étant tout particulièrement préférés, et l'on effectue la réaction d'abord à une température de -20° à -10°C, de préférence -15°C, puis en laissant remonter la température à 0°/+10°C, de préférence +5°C.

Des procédés conduisant au composé de formule (I) ont déjà été décrits. Ils consistent en général soit à traiter un composé 17-céto dont la double liaison 9(11) est déjà formée, par un cyanure alcalin dans un mélange acide acétique/alcanol, ou par la cyanhydrine de l'acétone en présence d'hydroxyde de sodium et d'eau (voir par exemple le brevet US 4.548.748, les brevets japonais 8262299 ou 8262300 - C. Abst. 97 141141-142 ou encore Bull. Chem. Soc. Japan 58 (3), 978 (1985)), soit à déshydrater un composé 9α,17α-dihydroxy 17β-cyano par l'acide sulfurique ou le complexe BF₃ Et₂O (voir par exemple le brevet US 4.921.638 ou la demande de brevet européen 263569, ou encore Stéroïds 55(3), 109 (1990)).

Ces procédés de déshydratation utilisant l'acide sulfurique ne peuvent guère être envisagés au niveau industriel en raison du faible rendement qu'ils procurent, une partie du produit de départ étant transformée en amide. Quant au procédé utilisant le complexe BF₃ Et₂O, il conduit certes à un bon rendement, mais il ne peut lui non plus être utilisé au niveau industriel, cette fois en raison de la quantité très importante de complexe BF₃ Et₂O qu'il faudrait mettre en oeuvre (voir p. 111 "méthode D" de la réf. Steroïds citée plus haut), 2 parties en poids de complexe par rapport au stéroïde mis en oeuvre étant en effet nécessaire, condition qui ajoutée au prix de ce réactif, est absolument rédhibitoire.

Par ailleurs, l'acide chlorosulfonique a déjà été utilisé comme agent de déshydratation en série stéroïde pour préparer des composés Δ9(11) à partir des composés 9α-OH correspondants (voir le brevet français 2 387 245).

Toutefois, il convient de remarquer, et ceci est essentiel, que la réaction est effectuée sur un seul type particulier de composés à savoir des composés comportant pour seuls substituants des groupements oxo (3 et 17) ou des radicaux méthyle ou fluoro, c'est-à-dire des composés ne comportant pas d'autre substituant hydroxy, et ne pouvant donc pas donner lieu à des réactions de compétitions entre la déshydratation en 9(11), qui peut elle-même d'ailleurs être en compétition avec une déshydratation en 8(9), et la déshydratation au niveau du site porteur de l'autre substituant hydroxy. Le composé de formule (I) utilisé au départ du procédé de la présente invention comporte cet autre substituant hydroxy en position 17α. Or, on sait notamment par la demande européenne n° 263 569 ou par l'article de stéroïds déjà cité, que dans de tels cas, l'utilisation d'un réactif de déshydratation du même type, à savoir le chlorure de méthane sulfonyle, conduit sélectivement, via un 17α-sulfonate intermédiaire, au dérivé Δ16(17) et non au Δ9(11) ou à un mélange des deux. On peut donc en conclure que l'obtention sélective selon l'invention, avec un excellent rendement, du dérivé Δ9(11) n'était pas prévisible, l'art antérieur enseignant qu'on devait plutôt s'attendre à obtenir le dérivé Δ16(17), ou tout au moins un mélange Δ9(11) et Δ16(17). Il est certain que d'un point de vue industriel, il est indispensable d'obtenir sélectivement et avec un rendement élevé le dérivé souhaité Δ9(11).

Le procédé de l'invention tient sa sélectivité des conditions dans lesquelles il est conduit. Le sulfonate est d'abord formé sélectivement à une température appropriée, la remontée en température étant ensuite nécessaire à l'élimination conduisant à la formation de la double liaison Δ9(11).

Il permet d'éviter la formation de la double liaison Δ16(17) dont on a vu plus haut qu'elle est aisée.

Le composé de formule (I) décrit dans la demande européenne n° 263 569, est un intermédiaire utile en particulier dans la synthèse de corticostéroïdes. Le composé de départ de formule (II) est également décrit dans cette demande.

L'exemple suivant illustre l'invention sans la limiter.

### EXEMPLE : 17β-cyano 17α-hydroxy androsta 4,9(11)-dièn-3-one

On mélange sous gaz inerte anhydre, 2 g de 17β-cyano 9α,17α-dihydroxy androst-4-èn-3-one et 10 cm³ de tétrahydrofuranne, puis refroidit à -15°C et introduit à cette température en 1 h, 0,5 cm³ d'acide chlorosulfonique. On maintient sous agitation et laisse la température remonter à +5°C en 30 mn, puis maintient à cette température pendant 1 h 30 mn. On ajoute alors 10 g de glace et 10 cm³ d'eau glacée, puis, après 30 mn, à nouveau 10 cm³ d'eau glacée. On agite pendant 1 h à 0°C, puis essore et lave les cristaux à l'eau puis les sèche. On obtient 1,72 g de produit attendu. F = 247°C (Rdt : 91 %). A partir des liqueurs mères, il est possible d'isoler à nouveau environ 3 % de produit attendu supplémentaire.
Spectre IR (CHCl₃)
Absorptions à 3620 cm⁻¹ (OH), 3569 cm⁻¹ (ép.) 1663 cm⁻¹ (>C=O), 1614 cm⁻¹ (C=C), 2238 cm⁻¹ (-C≡N).
Spectre RMN (CDCl₃, 250 MHz, ppm)
0,95 : H de CH₃ en 13 ; 1,36 : H de CH₃ en 10 ; 5,59 : H en 11 ; 5,72 : H en 4 ; 7,57 H de OH.

## Revendications

1. Procédé de préparation du composé de formule (I) : 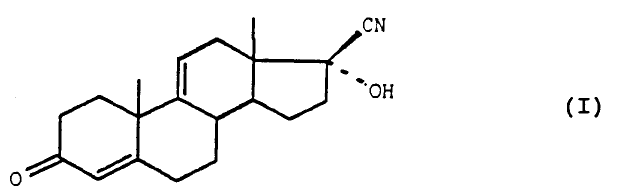 à partir d'un composé de formule (II): 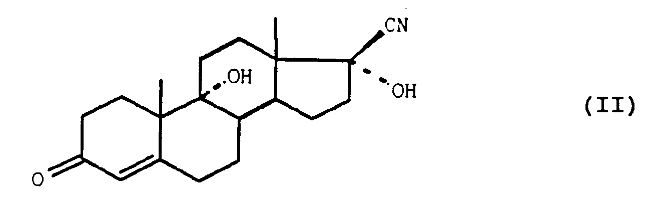 caractérisé par le fait que l'on traite le composé de formule (II) par l'acide chlorosulfonique au sein d'un solvant organique.

2. Procédé selon la revendication 1, caractérisé en ce que l'on opère au sein d'un solvant aprotique polaire choisi dans le groupe constitué par le tétrahydrofuranne, le dioxanne et l'acétone, ou de l'acétate d'éthyle ou du chlorure de méthylène.

3. Procédé selon la revendication 2, caractérisé en ce que l'on opère au sein du tétrahydrofuranne ou de l'acétone.

4. Procédé selon l'une quelconque des revendications 1 à 3, caractérisé en ce que l'on opère à une température de -20° à -10°C puis en laissant remonter à une température de 0°/+5°C.

5. Procédé selon la revendication 4, caractérisé en ce que l'on opère à une température de -15°C, puis en laissant remonter à une température de +5°C.

## Claims

1. Preparation process for the compound of formula (I): 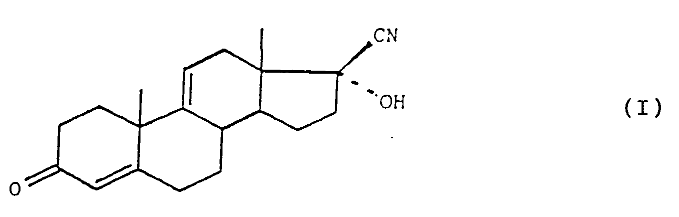 starting from a compound of formula (II): 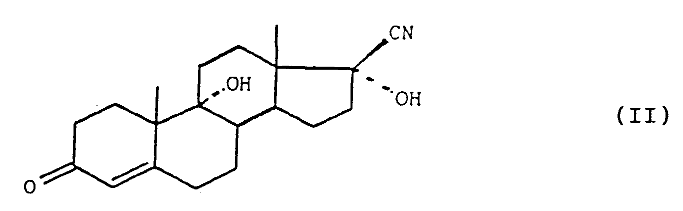 characterized in that the compound of formula (II) is treated with chlorosulphonic acid in an organic solvent.

2. Process according to claim 1, characterized in that the operation takes place in an aprotic polar solvent chosen from the group constituted by tetrahydrofuran, dioxane and acetone, or ethyl acetate or methylene chloride.

3. Process according to claim 2, characterized in that the operation takes place in tetrahydrofuran or acetone.

4. Process according to any one of claims 1 to 3, characterized in that the operation is carried out at a temperature of -20° to -10°C then the reaction medium is left to rise to a temperature of 0°/+5°C.

5. Process according to claim 4, characterized in that the operation is carried out at a temperature of -15°C, then the reaction medium is left to rise to a temperature of +5°C.

## Patentansprüche

1. Verfahren zur Herstellung einer Verbindung der Formel (I) 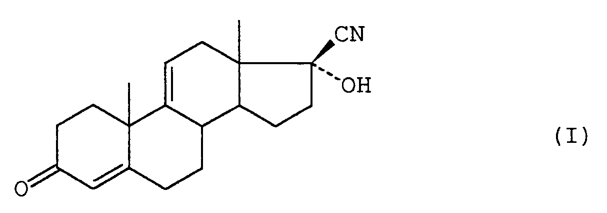 ausgehend von einer Verbindung der Formel (II) 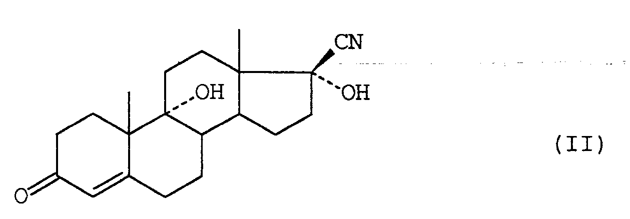 dadurch gekennzeichnet, daß man die Verbindung der Formel (II) in einem organischen Lösungsmittel mit Chlorsulfonsäure behandelt.

2. Verfahren nach Anspruch 1, dadurch gekennzeichnet, daß man in einem polaren aprotischen Lösungsmittel arbeitet, gewählt aus der durch Tetrahydrofuran, Dioxan und Aceton, oder Ethylacetat oder Methylenchlorid gebildeten Gruppe.

3. Verfahren nach Anspruch 2, dadurch gekennzeichnet, daß man in Tetrahydrofuran oder Aceton arbeitet.

4. Verfahren nach irgendeinem der Ansprüche 1 bis 3, dadurch gekennzeichnet, daß man bei einer Temperatur von -20 °C bis -10 °C arbeitet und anschließend wieder auf eine Temperatur von 0 °C/+5 °C ansteigen läßt.

5. Verfahren nach Anspruch 4, dadurch gekennzeichnet, daß man bei einer Temperatur von -15 °C arbeitet und anschließend wieder auf eine Temperatur von +5 °C ansteigen läßt.
